# EUROPÄISCHE PATENTSCHRIFT

(11) **EP 2 386 258 B1**
(45) Veröffentlichungstag und Bekanntmachung des Hinweises auf die Patenterteilung: **22.06.2016**
(21) Anmeldenummer: 11161088.7
(22) Anmeldetag: 05.04.2011
(51) Int. Cl.: A61B 17/70

(54) **Haltevorrichtung für Wirbelkörper der Wirbelsäule**
Holding device for spine vertebrae
Dispositif de retenue pour vertèbres de la colonne vertébrale

(30) Priorität: 10.05.2010 DE 102010016854
(43) Veröffentlichungstag der Anmeldung: 16.11.2011
(73) Patentinhaber: Ulrich GmbH & Co. KG, 89081 Ulm (DE)
(72) Erfinder: Kast, Erich, 8307 Effretikon (CH)
(74) Vertreter: Hentrich Patentanwälte PartG mbB

(56) Entgegenhaltungen:
- FR-A1- 2 924 014
- US-A1- 2010 030 268
- US-A1- 2010 030 271

## Beschreibung

Die Erfindung betrifft eine Haltevorrichtung für Wirbelkörper der Wirbelsäule aufweisend ein in einen Wirbelkörper einzubringendes Implantat und einen Pedikelstab, wobei das Implantat für die Beweglichkeit des Pedikelstabes einen Hohlraum aufweist, der Pedikelstab in dem Hohlraum für eine permanente Beweglichkeit des Pedikelstabes in Bezug auf das Implantat angeordnet ist und das Implantat eine hülsenförmige Ausgestaltung aufweist.

Bei einer herkömmlichen Haltevorrichtung für Wirbelkörper der Wirbelsäule ist der Pedikelstab starr in dem Implantat angeordnet, wobei über Fixiervorrichtungen an einen Verbindungstab zwischen zwei Pedikelstäben deren Ausrichtung festgestellt werden kann. Diese Ausgestaltung weist den Nachteil auf, dass die Stabilisierung der Wirbelkörper starr ist, wodurch der Bewegungsumfang eingeschränkt wird.

Eine Haltevorrichtung der eingangs genannten Art ist aus der US 2010 003 0268 A1 bekannt, die eine Pedikelschraube zeigt, deren Kopplung mit einem Verbindungselement nicht über die bekannte Tulpenkopfaufnahme, sondern über eine nach Art einer Spannzange gebildete Spannvorrichtung erfolgt.

Es kann eine feste, aber lösbare Verbindung zwischen Implantat und Pedikelstab erreicht werden.

Der Erfindung liegt daher die Aufgabe zugrunde, eine Haltevorrichtung für Wirbelkörper der Wirbelsäule der eingangs genannten Art so auszubilden, dass die Nachteile der herkömmlichen Haltevorrichtung hinsichtlich des Bewegungsumfanges überwunden werden können.

Gemäß der vorliegenden Erfindung wird diese Aufgabe bei der eingangs genannten Haltevorrichtung dadurch gelöst, dass das Implantat eine Gewindeverankerung in dem Hohlraum und der Pedikelstab einen in die Gewindeverankerung einbringbaren Gewindeabschnitt aufweist, und das Implantat posterior auf einer Seite einen Verlängerungsabschnitt aufweist, der für eine dauerhafte Anbringung eines mit einer zweiten Haltevorrichtung verbundenen Verbindungselements mit einem Kopplungsglied ausgestaltet ist. Diese Ausgestaltung weist den Vorteil auf, dass eine dynamische segmentale Stabilisierung mit einem Rotationszentrum im Bereich des physiologischen Drehzentrums ventral von der Pedikelebene erreicht werden kann. Dadurch wird ein weitgehend physiologisches Bewegungsmuster bewirkt. Ein zusätzlicher Vorteil liegt darin, dass ein effektiver Schutz der Anschlussdegeneration und die Erhaltung eines größeren Bewegungsumfanges erreicht werden kann.

Weiterhin weist das Implantat eine hülsenförmige Ausgestaltung auf, wodurch die Einbringung des Implantates in den Wirbelkörper erleichtert wird.

Bevorzugt weist der Pedikelstab einen elastischen Bereich auf. Besonders bevorzugt ist der elastische Bereich aus einem Material gebildet, das aus einer Gruppe ausgewählt worden ist, die umfasst: Metalle und insbesondere Titan und dessen Legierungen, Stahl oder CoCr oder Kunststoffe und insbesondere Polyethylen, Polyurethan, Polyetheretherketon, kohlenstofffaserverstärkten Kunststoff oder superelastischen Nitinol Diese Ausgestaltung weist den Vorteil auf, dass die Beweglichkeit des Pedikelstabes zu dem Implantat einfach bewirkt werden kann.

Weiterhin kann das Implantat ein elastisches Element aufweisen, das an einem Verbindungsbereich zwischen dem Implantat und dem Pedikelstab angeordnet ist.

Diese Ausführung weist den Vorteil auf, dass durch die Verwendung eines dämpfenden Elementes eine zusätzliche Stabilisierung erreicht werden kann. Hierfür kann z. B. ein Hohlzylinder aus Polycarbonaturethan (PCU) in dem Hohlraum zwischen Implantat und Pedikelstab angebracht werden, wobei die Form und die Geometrie variiert werden kann, um das Ausmaß der Dämpfung und der Stabilisierung gezielt zu bestimmen.

Außerdem kann eine einachsige, eine zweiachsige oder eine polyaxiale Gelenkverbindung zwischen dem Implantat und dem Pedikelstab vorgesehen sein, wodurch die Beweglichkeit, insbesondere die Flexion und die Extension, des Pedikelstabes zu dem Implantat einfach bewirkt werden kann.

Bei der Haltevorrichtung für Wirbelkörper hat es sich als vorteilhaft erwiesen, wenn in dem posterioren Bereich das Implantat eine Anschlagfläche und der Pedikelstab eine Anschlagsgegenfläche zur Begrenzung der Beweglichkeit zwischen dem Implantat und dem Pedikelstab aufweisen.

Bevorzugt weist der Pedikelstab posterior eine schließbare Stabaufnahme auf. In diesem Zusammenhang hat es sich als besonders günstig erwiesen, wenn die Stabaufnahme ein verstellbares Sperrelement umfasst. Diese Ausgestaltung weist den Vorteil auf, dass die Herstellung einer winkelstabilen Verbindung sekundär über einen perkutanen Eingriff möglich wird und z. B. bei kritischen Knochenverhältnissen ein Einwachsen der Implantate ohne Belastung ermöglicht wird. Wenn das Einwachsen erfolgt ist, kann z.B. perkutan in Lokalanästhesie die dorsale Verbindung geschlossen werden, wodurch die dynamische oder auch rigide segmentale Stabilisierung aktiviert wird.

Bei einer Haltevorrichtung für Wirbelkörper nach der vorliegenden Erfindung ist es vorteilhaft, wenn der Pedikelstab einen ovalen Querschnitt aufweist. Durch den ovalen Querschnitt kann der Bewegungsumfang für Flexionen und/oder Extensionen bei gleichbleibender Stabilität erhöht werden. Aufgrund des ovalen Querschnittes kann das Widerstandsmoment in unterschiedliche Ebenen unterschiedlich groß sein, so dass z.B. die Flexion leichter möglich ist als die Seitbiegung. Weiterhin wird hierdurch die Stabilität bei Rotationen erhöht.

Besonders vorteilhaft ist es, wenn das Implantat mit einer posterior abnehmenden Wandstärke ausgebildet ist.

Erfindungsgemäß weist bei der Haltevorrichtung für Wirbelkörper das Implantat posterior auf einer Seite einen Verlängerungsabschnitt auf, der für eine dauerhafte Anbringung eines mit einer zweiten Haltevorrichtung verbundenen Verbindungselements mit einem Kopplungsglied, insbesondere mit einer Verbindungselementaufnahme ausgestaltet ist. Diese Ausgestaltung ist für mehrsegmentale dynamische Anwendungen besonders geeignet. Zum Erreichen einer vollen Dynamik, insbesondere bei Flexion/Extension, über mehrere Segmente ist es erforderlich, die hierbei im Bereich der dorsalen Verbindungselemente auftretenden Längenänderungen zu kompensieren, so dass sich die Beweglichkeiten der einzelnen Segmente summieren können. Die Verbindung zwischen den einzelnen dynamischen

Haltevorrichtungen erfolgt so, dass jeweils der Pedikelstab der einen Haltevorrichtung mit dem nächst kranialeren Implantat (Hülse) an dem Verlängerungsabschnitt über das Kopplungsmittel verbunden wird. Dadurch kann eine beliebig lange Stabilisierung erreicht werden. Dieser Vorteil kann nach der vorliegenden Erfindung für ein dorsales Fixationssystem mit einer Anzahl an derartigen Haltevorrichtungen dadurch erreicht werden, dass mindestens die erste Haltevorrichtung ein Verbindungselement aufweist, das über ein Befestigungsteil der ersten Haltevorrichtung mit dem Pedikelstab der ersten Haltevorrichtung und über das Kopplungsmittel der zweiten Haltevorrichtung mit der zweiten Haltevorrichtung verbunden ist.

Nach einem zweiten Gesichtspunkt der vorliegenden Erfindung wird diese Aufgabe bei einem dorsalem Fixationssystem dadurch gelöst, dass eine erste Haltevorrichtung für Wirbelkörper nach einem der oben genannten Ausführungsbeispiele und eine zweite Haltevorrichtung für Wirbelkörper mit einem Verbindungselement vorgesehen ist, das ein erstes Befestigungsteil mit dem ersten Pedikelstab der ersten Haltevorrichtung und ein zweites Befestigungsteil mit dem zweiten Pedikelstab der zweiten Haltevorrichtung aufweist. Diese Ausgestaltung weist den Vorteil auf, dass durch das physiologischer platzierte Drehzentrum im dorsalen Anteil des Wirbelkörpers eine bessere Segmentbeweglichkeit bewirkt wird. Dadurch kann das System z. B. auch als eine Bandscheibenprothese verwendet werden. Darüber hinaus ist in einem späteren Stadium auch eine Kombination mit einem Facettengelenkersatz möglich.

Bevorzugt sind bei dem dorsalen Fixationssystem das erste Befestigungsteil und/oder das zweite Befestigungsteil ausgestaltet, einen ersten Winkel zwischen dem Verbindungselement und dem ersten Pedikelstab der ersten Haltevorrichtung bzw. einen zweiten Winkel zwischen dem Verbindungselement und dem zweiten Pedikelstab der zweiten Haltevorrichtung stabil zu halten.

Alternativ kann bei dem dorsalen Fixationssystem das erste Befestigungsteil und/oder das zweite Befestigungsteil ausgestaltet sein, einen ersten Winkel zwischen dem Verbindungselement und dem ersten Pedikelstab der ersten Haltevorrichtung bzw. einen zweiten Winkel zwischen dem Verbindungselement und dem zweiten Pedikelstab der zweiten Haltevorrichtung variabel zu halten.

Bei dem dorsalen Fixationssystem hat es sich als vorteilhaft erwiesen, wenn das Verbindungselement einen teleskopischen Bereich und/oder einen flexiblen Bereich aufweist, um die im Bereich der dorsalen Verbindungselemente auftretenden Längenänderungen zu kompensieren.

Bevorzugt weist das dorsale Fixationssystem eine dritte Haltevorrichtung für Wirbelkörper auf und ist das Verbindungselement über ein drittes Befestigungsteil an der dritten Haltevorrichtung mit einem dritten Pedikelstab der dritten Haltevorrichtung verbunden ist. Auf diese Weise kann eine mehrsegmentale dynamische Stabilisierung erreicht werden.

Ein weiterer Vorteil des erfindungsgemäßen Fixationssystems liegt darin, dass über das dorsale Verbindungselement eine Segmentdistraktion möglich ist, um z. B. die Bandscheibe kontinuierlich zu entlasten.

Im Folgenden wird die Erfindung an in den Zeichnungen dargestellten Ausführungsbeispielen näher erläutert; es zeigen:
- Fig. 1: eine Querschnittsdarstellung einer Haltevorrichtung für Wirbelkörper der Wirbelsäule nach einem ersten Ausführungsbeispiel der vorliegenden Erfindung,
- Fig. 2: eine Querschnittsdarstellung einer Haltevorrichtung für Wirbelkörper der Wirbelsäule nach einem zweiten Ausführungsbeispiel der vorliegenden Erfindung,
- Fig. 3: eine Querschnittsdarstellung einer Haltevorrichtung für Wirbelkörper der Wirbelsäule nach einem dritten Ausführungsbeispiel der vorliegenden Erfindung,
- Fig. 4: eine Querschnittsdarstellung einer Haltevorrichtung für Wirbelkörper der Wirbelsäule nach einem vierten Ausführungsbeispiel der vorliegenden Erfindung,
- Fig. 5: eine Querschnittsdarstellung ähnlich der Fig. 1 bis 4 mit unterbundenen Freiheitsgraden entsprechend einer herkömmlichen Haltevorrichtung,
- Fig. 6: eine Querschnittsdarstellung eines Pedikelstabes mit einem posterioren Verbindungselement,
- Fig. 7: eine Querschnittsdarstellung eines dorsalen Fixationssystems mit einer ersten Haltevorrichtung für Wirbelkörper der Wirbelsäule und einer zweiten Haltevorrichtung für Wirbelkörper der Wirbelsäule nach einem ersten Ausführungsbeispiel der vorliegenden Erfindung,
- Fig. 8: eine Querschnittsdarstellung eines dorsalen Fixationssystems für mehrsegmentale Anwendungen nach einem zweiten Ausführungsbeispiel,
- Fig. 9: eine Querschnittsdarstellung eines dorsalen Fixationssystems mit einem flexiblen Verbindungselement nach einem dritten Ausführungsbeispiel,
- Fig. 10: eine schematische Darstellung der Bewegungsmöglichkeiten eines dorsalen Fixationssystems,
- Fig. 10a: eine gegenüber Figur 10 weiter abstrahierte Darstellung des dorsalen Fixationssystems in Ruhezustand,
- Fig. 10b: eine der Figur 10a entsprechenden Darstellung im aufgrund von Bewegung ausgelenktem Zustand,
- Fig. 11: eine Querschnittsdarstellung eines dorsalen Fixationssystems nach einem vierten Ausführungsbeispiel,
- Fig. 12: eine Querschnittsdarstellung eines dorsalen Fixationssystems nach einem fünften Ausführungsbeispiel, und
- Fig. 13: eine Querschnittsdarstellung eines dorsalen Fixationssystems nach einem sechsten Ausführungsbeispiel.

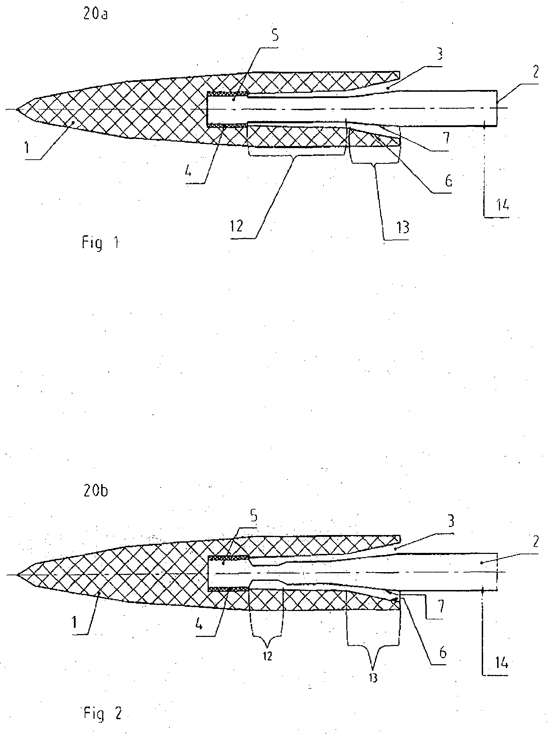

Fig. 1 zeigt eine Haltevorrichtung 20a für Wirbelkörper der Wirbelsäule nach einem ersten Ausführungsbeispiel der vorliegenden Erfindung. Die Haltevorrichtung 20a umfasst ein Implantat 1 mit einem Hohlraum 3 und einen Pedikelstab 2. Das Implantat 1 weist eine Gewindeverankerung 4 auf, in die ein Gewindeabschnitt 5 des Pedikelstabes 2 eingebracht werden kann, wobei sich ein Durchmesser von 4 mm bis 5 mm für den Gewindeabschnitt 5 des Pedikelstabes 2 als vorteilhaft erwiesen hat. Weiterhin kann das Implantat 1 eine konische Grundform mit einem Außengewinde aufweisen, wobei der vordere Gewindeanteil stumpf ausgestaltet sein kann, um eine bikortiale Verankerung zu ermöglichen. In dem hinteren Bereich kann das Außengewinde zunehmend flacher und schneidend werden, um ein Sprengen des Pedikels zu vermeiden. Vorzugsweise ist die Oberfläche des Implantates beschichtet. Die Wandstärke des Implantates 1 kann posterior abnehmend ausgestaltet sein. Das Implantat 1 kann von posterior transpedikulär in die lumbalen Wirbelkörper eingebracht und verankert werden, wobei sich eine hülsenförmige Ausgestaltung des Implantates 1 als vorteilhaft erwiesen hat. Die Verankerung im Wirbelkörper kann über die Oberflächenstruktur (wie z. B. Gewinde oder Beschichtung) und/oder über eine Formänderung des Implantates 1 (wie z. B. Spreizung, Stauchung oder Ballonierung) erfolgen.

Der Pedikelstab 2 weist weiterhin einen dynamischen Bereich 12, einen Verankerungsbereich 14 und einen Bewegungslimitierungsbereich 13 auf, bei dem die Anlage einer Anschlagsgegenfläche 7 des Pedikelstabes 2 auf eine Anschlagsfläche 6 des Implantates 1 die Bewegung begrenzt. Somit sind das Implantat 1 und der Pedikelstab 2 im posterioren Bereich so geformt, dass eine Bewegungslimitierung in dem gewünschten Rahmen erfolgt. Um den zulässigen Bewegungsumfang gezielt zu bestimmen, können entsprechende Ausgestaltungen der Anschlagsfläche 6 des Implantates 1 und der Anschlagsgegenfläche 6 des Pedikelstabes 2 gewählt werden.

Um das Ausmaß der Dynamik und das Drehzentrum besser an patientenspezifische Anforderungen anpassen zu können, kann der Pedikelstab 2 in dem dynamischen Bereich 12 verschiedene Durchmesser und/oder unterschiedliche Ausgestaltungen aufweisen. Bei dem in der Fig. 1 gezeigten Haltevorrichtung 20a nach dem ersten Ausführungsbeispiel weist der Pedikelstab 2 in dem dynamischen Bereich 12 einen im Vergleich zu dem Gewindeabschnitt 5 im Wesentlichen gleichen Durchmesser auf, wobei in dem ersten Ausführungsbeispiel der dynamische Bereich 12 des Pedikelstabes 2 an den Gewindeabschnitt 5 des Pedikelstabes 2 angrenzend angeordnet ist. Bei der Beschreibung der weiteren Ausführungsbeispiele der nachfolgenden Figuren ist zu beachten, dass gleiche Elemente mit gleichen Bezugszeichen versehen sind und dass zur Vermeidung von Wiederholungen für die folgenden Ausführungsbeispiele nur auf die Unterschiede eingegangen wird.

Fig. 2 zeigt eine Haltevorrichtung 20b für Wirbelkörper der Wirbelsäule nach einem zweiten Ausführungsbeispiel der vorliegenden Erfindung, bei dem der Pedikelstab 2 in dem dynamischen Bereich 12 einen im Vergleich zu dem Gewindeabschnitt 5 verkleinerten Durchmesser aufweist, wobei in dem zweiten Ausführungsbeispiel der dynamische Bereich 12 des Pedikelstabes 2 an den Gewindeabschnitt 5 des Pedikelstabes 2 angrenzend angeordnet ist.

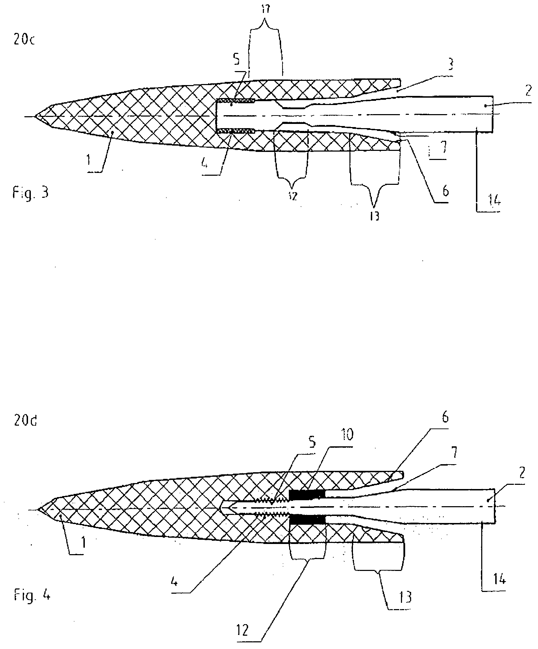

Fig. 3 zeigt eine Haltevorrichtung 20c für Wirbelkörper der Wirbelsäule nach einem dritten Ausführungsbeispiel der vorliegenden Erfindung, bei dem der der Pedikelstab 2 in dem dynamischen Bereich 12 einen im Vergleich zu dem Gewindeabschnitt 5 verkleinerten Durchmesser aufweist, wobei in dem dritten Ausführungsbeispiel zwischen dem dynamische Bereich 12 des Pedikelstabes 2 und dem Gewindeabschnitt 5 des Pedikelstabes 2 ein an dem Implantat 1 anliegender Verstärkungsbereich 17 mit vergrößerten Durchmesser angeordnet ist.

Fig. 4 zeigt eine Haltevorrichtung 20d für Wirbelkörper der Wirbelsäule nach einem vierten Ausführungsbeispiel der vorliegenden Erfindung, bei dem der Pedikelstab 2 in dem dynamischen Bereich 12 einen im Vergleich zu dem Gewindeabschnitt 5 verkleinerten Durchmesser aufweist, wobei in dem vierten Ausführungsbeispiel der dynamische Bereich 12 des Pedikelstabes 2 an dem Gewindeabschnitt 5 des Pedikelstabes 2 angrenzend angeordnet ist und wobei zusätzliche ein elastisches Element 10 zwischen dem dynamischen Bereich 12 des Pedikelstabes 2 und dem Implantat 1 angeordnet ist.

Durch unterschiedliche Ausgestaltungen des dynamischen Bereiches kann somit eine optimale Adaption an die speziellen Bedürfnisse der jeweiligen Patienten erfolgen. Weiterhin kann ein rigider Pedikelstab in dem System enthalten sein, um einen sekundären Wechsel von einer dynamischen Stabilisierung zu einer rigiden Stabilisierung zu ermöglichen oder um einen sekundären Wechsel von einer rigiden Stabilisierung zu einer dynamischen Stabilisierung zu ermöglichen.

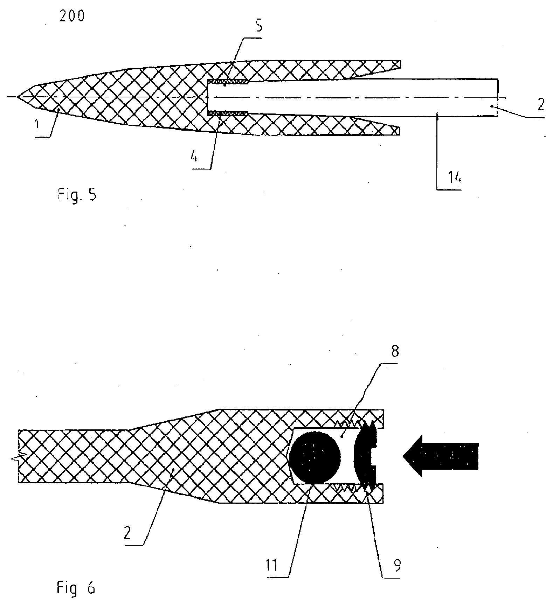

Zum besseren Verständnis der vorliegenden Erfindung ist in der Fig. 5 eine herkömmliche Haltevorrichtung 200 für Wirbelkörper der Wirbelsäule gezeigt. Die herkömmliche Haltevorrichtung 200 umfasst ein Implantat 1 und einen Pedikelstab 2, wobei weder ein dynamischer Bereich noch ein Bewegungslimitierungsbereich ausgebildet sind, so dass nur eine starre Verankerung erfolgen kann.

Fig. 6 zeigt eine Querschnittsdarstellung eines Pedikelstabes 2 mit einem posterioren Verbindungselement 11 für die Verbindung mit einem weiteren Pedikelstab, wobei das Verbindungselement 11 vorzugsweise als ein Verbindungstab ausgebildet ist. Der Pedikelstab 2 weist posterior eine schließbare Stabaufnahme 8 für das Verbindungselement 11 auf. Zur Aktivierung der Verbindung kann ein Sperrelement 9 vorgesehen sein, das zur Aktivierung der Verbindung in die in der Fig. 6 dargestellte Pfeilrichtung bewegt werden kann.

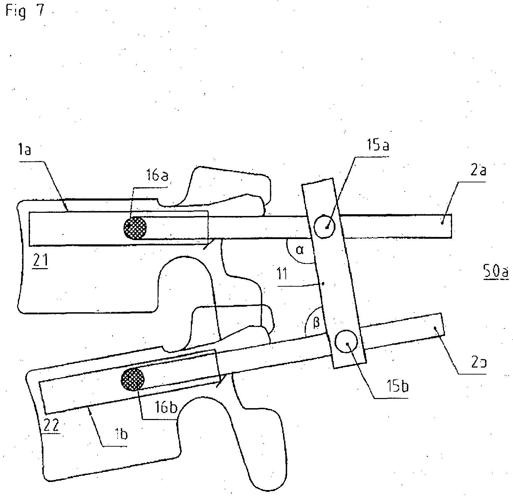

Fig. 7 zeigt eine Querschnittsdarstellung eines dorsalen Fixationssystems 50 mit einer ersten Haltevorrichtung 21 für Wirbelkörper der Wirbelsäule nach der vorliegenden Erfindung und einer zweiten Haltevorrichtung 22 für Wirbelkörper der Wirbelsäule nach der vorliegenden Erfindung mit einem Verbindungselement 11, das ein erstes Befestigungsteil 15a für eine Befestigung mit einem ersten Pedikelstab 2a der ersten Haltevorrichtung 21 und das ein zweites Befestigungsteil 15b für eine Befestigung mit einem zweiten Pedikelstab 2b der zweiten Haltevorrichtung 22 aufweist. Alternativ ist es bei dem dorsalen Fixationssystem nach der vorliegenden Erfindung auch möglich, dass die erste Haltevorrichtung 21 oder die zweite Haltevorrichtung 22 durch eine in der Fig. 7 nicht gezeigte herkömmliche Haltevorrichtung für Wirbelkörper der Wirbelsäule mit einem starren Pedikelstab ersetzt wird. Auf diese Weise kann eine Kombination von rigider und dynamischer Stabilisierung erreicht werden.

Weiterhin weist bei dem in der Fig. 7 gezeigten Ausführungsbeispiel das dorsale Fixationssystem 50 in der ersten Haltevorrichtung 21 ein erstes Drehzentrum 16a und in der zweiten Haltevorrichtung 22 ein zweites Drehzentrum 16b auf. Das erste Befestigungsteil 15a und/oder das zweite Befestigungsteil 15b können ausgestaltet sein, einen ersten Winkel α zwischen dem Verbindungselement 11 und dem ersten Pedikelstab 2a der ersten Haltevorrichtung 21 bzw. einen zweiten Winkel β zwischen dem Verbindungselement 11 und dem zweiten Pedikelstab 2b der zweiten Haltevorrichtung 22 stabil zu halten. Alternativ kann das erste Befestigungsteil 15a und/oder das zweite Befestigungsteil 15b ausgestaltet sein, den ersten Winkel α zwischen dem Verbindungselement 11 und dem ersten Pedikelstab 2a der ersten Haltevorrichtung 21 bzw. den zweiten Winkel β zwischen dem Verbindungselement 11 und dem zweiten Pedikelstab 2b der zweiten Haltevorrichtung 22 variabel zu halten.

Weiterhin ist es bei dem dorsalen Fixationssystem nach der vorliegenden Erfindung die Anzahl der Haltevorrichtungen für Wirbelkörper der Wirbelsäule nicht auf zwei begrenzt, sondern es können auch mehr Haltevorrichtungen eingesetzt werden.

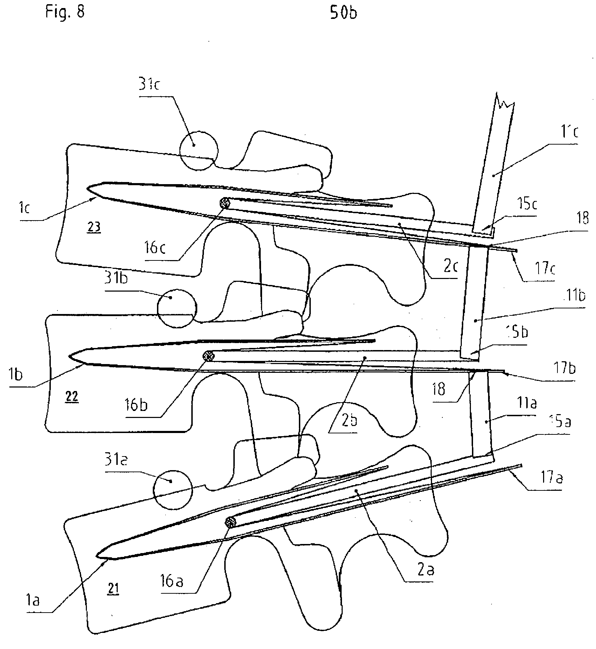

Fig. 8 zeigt eine Querschnittsdarstellung eines dorsalen Fixationssystems 50b für mehrsegmentale Anwendungen nach einem zweiten Ausführungsbeispiel mit drei Haltevorrichtungen 21, 22, 23. Die Haltevorrichtungen 21, 22, 23 weisen jeweils einen Pedikelstab 2a, 2b, 2c auf, an dem jeweils ein Verbindungselement 11 a, 11 b, 11 c über ein entsprechendes Befestigungsteil 15a, 15b, 15c angebracht ist. Weiterhin sind das erste und das zweite Verbindungselement 11a, 11b mit dem nächst kranialerem Implantat durch Kopplungsmittel 18 an den posterioren Verlängerungsabschnitten 17 der entsprechenden Implantate 1 verbunden. Bei diesem Ausführungsbeispiel befinden sich die physiologischen Drehzentren 31 a, 31 b, 31 c in der Nähe der mechanischen Drehzentren 16a, 16b, 16c.

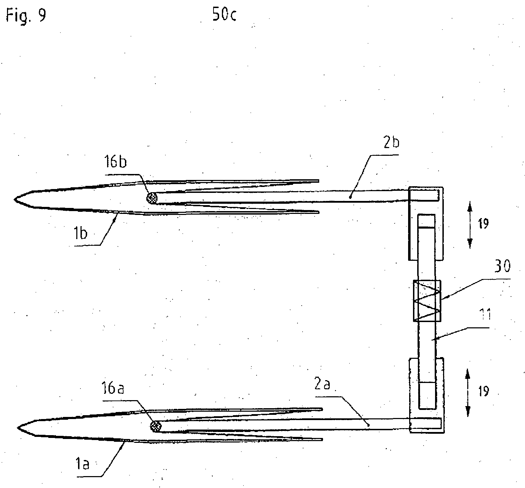

Fig. 9 zeigt eine Querschnittsdarstellung eines dorsalen Fixationssystems 50c mit einem flexiblen Verbindungselement nach einem dritten Ausführungsbeispiel. Der Aufbau ähnelt dem in Fig. 7 gezeigten Ausführungsbeispiel, wobei das Verbindungselement 11 einen teleskopischen Bereich 19 und einen flexiblen Bereich 30 aufweist, der in diesem Fall durch eine Verjüngung in dem Verbindungselement 11 gebildet ist.

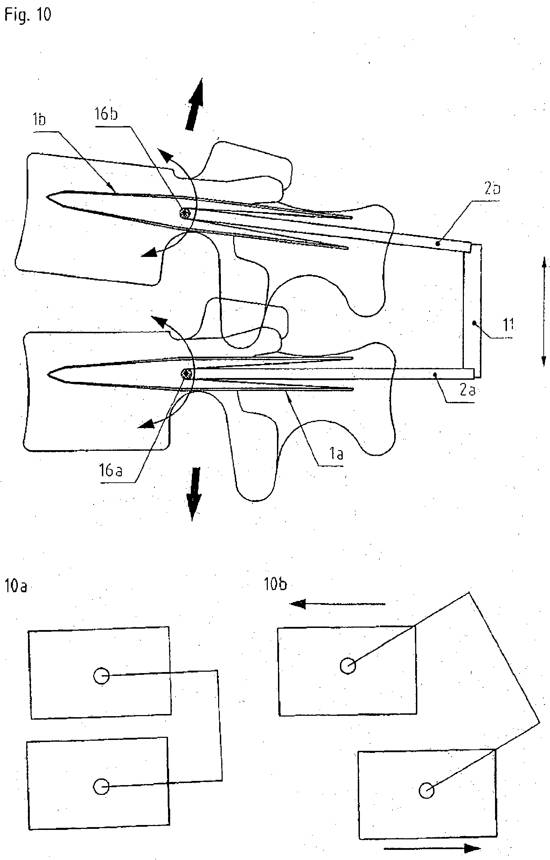

in der Fig. 10 wird eine schematische Darstellung der Bewegungsmöglichkeiten des dorsalen Fixationssystems bei monosegmentaler Instrumentierung gezeigt. Neben der durch Pfeile angedeuteten Drehbewegung ist auch eine Translation möglich. Darüber. hinaus können durch die Trennung vom Implantat und Pedikelstab mögliche Schraubenauslockerungen verringert werden, da die Bewegungen aus der Dynamik nicht über eine Knochen-Implantat-Schnittstelle, sondern über die Implantat-Pedikelstab-Schnittstelle übertragen werden. Weiterhin findet eine Distraktion dorsal über die Pedikelstäbe und den Längsträger statt, wodurch die Dynamik (Bewegungsumfang) erhalten bleibt. Außerdem kann eine Kyphosierung vermieden werden, so dass eine aktive Beeinflussung des saggitalen Profils ohne Beeinträchtigung des Bewegungsumfanges ermöglicht wird.

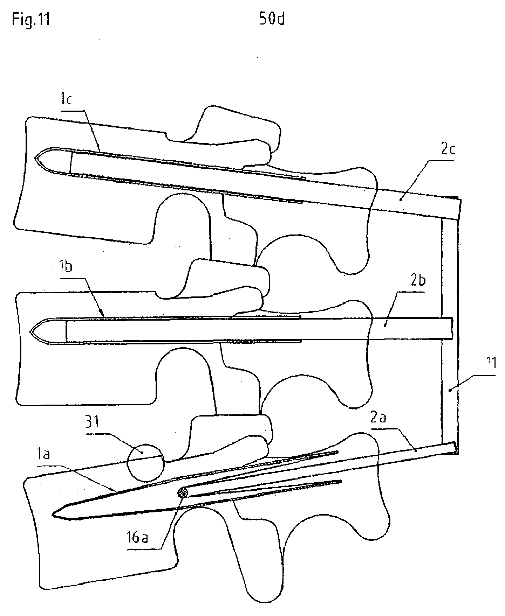

Fig. 11 zeigt eine Querschnittsdarstellung eines dorsalen Fixationssystems 50d nach einem vierten Ausführungsbeispiel, bei dem sich ein physiologisches Drehzentrum 31 caudal in der Nähe des mechanischen Drehzentrums 16 des Implantates befindet. Somit ist ein starres Verbindungselement 11 möglich und es können für die zweite und dritte Haltevorrichtung herkömmliche Haltevorrichtungen verwendet werden, da keine zusätzliche Beweglichkeit notwendig ist. Somit umfasst das dorsale Fixationssystem 50d nach dem vierten Ausführungsbeispiel eine erfindungsgemäße und zwei herkömmliche Haltevorrichtungen.

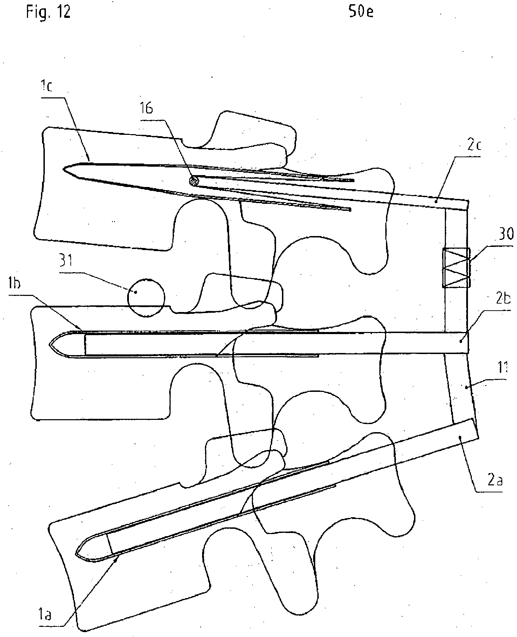

Fig. 12 zeigt eine Querschnittsdarstellung eines dorsalen Fixationssystems 50e nach einem fünften Ausführungsbeispiel, bei dem sich das physiologische Drehzentrum 31 weit entfernt vom mechanischen Drehzentrum 16 befindet, so dass eine zweite Bewegung notwendig wird. Dies erfordert ein dynamisches Verbindungselement 11. Wie in der Fig. 12 gezeigt ist, kann dies durch einen flexiblen Bereich 30 erreicht werden, der z. B. durch eine Verjüngung gebildet wird. Wiederum umfasst das dorsale Fixationssystem 50e nach dem fünften Ausführungsbeispiel eine erfindungsgemäße und zwei herkömmliche Haltevorrichtungen.

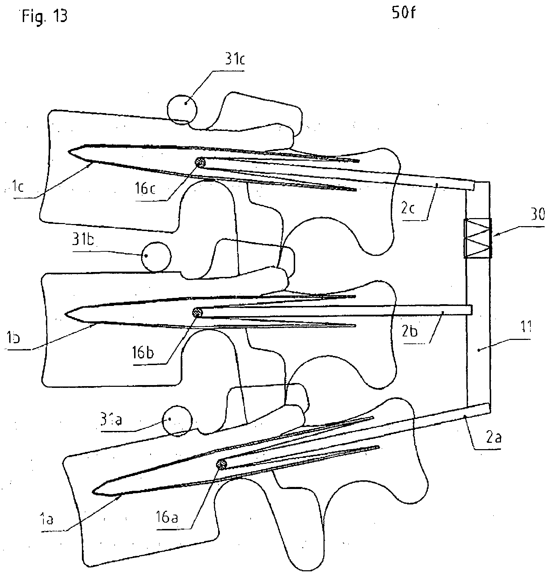

Fig. 13 zeigt eine Querschnittsdarstellung eines dorsalen Fixationssystems 50f nach einem sechsten Ausführungsbeispiel mit drei erfindungsgemäßen Haltevorrichtungen, bei dem sich die drei physiologischen Drehzentren 31 a, 31 b, 31 c caudal in der Nähe der drei mechanischen Drehzentren 16a, 16b, 16c befinden. Im Gegensatz zu einer monosegmentalen Instrumentierung, bei der zwei Bewegungsbereiche ausreichen, um Flexion und Translation zu bewirken, sind bei der hier vorliegenden mehrsegmentalen Instrumentierung weitere Bewegungsbereiche notwendig, die bei dem in der Fig. 13 vorliegenden Ausführungsbeispiel als Verjüngung ausgebildet sind.

### Bezugszeichenliste

- 1: Implantat
- 1a: erstes Implantat
- 1b: zweites Implantat
- 1c: drittes Implantat
- 2: Pedikelstab
- 2a: erster Pedikelstab
- 2b: zweiter Pedikelstab
- 2c: dritter Pedikelstab
- 3: Hohlraum
- 4: Gewindeverankerüng im Implantat
- 5: Gewindeabschnitt des Pedikelstabes
- 6: Anschlagfläche des Implantates
- 7: Anschlagsgegenfläche des Pedikelstabes
- 8: schließbare Stabaufnahme
- 9: Sperrelement
- 10: elastisches Element
- 11: Verbindungselement
- 11a: erstes Verbindungselement
- 11b: zweites Verbindungselement
- 11c: drittes Verbindungselement
- 12: dynamischer Bereich
- 13: Bewegungslimitierungsbereich
- 14: Verankerungsbereich
- 15a: erstes Befestigungsteil
- 15b: zweites Befestigungsteil
- 15c: drittes Befestigungsteil
- 16a: erstes mechanisches Drehzentrum
- 16b: zweites mechanisches Drehzentrum
- 16c: drittes mechanisches Drehzentrum
- 17a: erster posteriorer Verlängerungsabschnitt
- 17b: zweiter posteriorer Verlängerungsabschnitt
- 17c: dritter posteriorer Verlängerungsabschnitt
- 18: Kopplungsmittel
- 19: teleskopischer Bereich des Verbindungselementes
- 20a: Haltevorrichtung nach erstem Ausführungsbeispiel
- 20b: Haltevorrichtung nach zweitem Ausführungsbeispiel
- 20c: Haltevorrichtung nach drittem Ausführungsbeispiel
- 20d: Haltevorrichtung nach viertem Ausführungsbeispiel
- 21: erste Haltevorrichtung
- 22: zweite Haltevorrichtung
- 23: dritte Haltevorrichtung
- 31: physiologisches Drehzentrum
- 30: flexibler Bereich des Verbindungselementes
- 31a: erstes physiologisches Drehzentrum
- 31b: zweites physiologisches Drehzentrum
- 31c: drittes physiologisches Drehzentrum
- 50a: dorsales Fixationssystem nach einem ersten Ausführungsbeispiel
- 50b: dorsales Fixationssystem nach einem zweiten Ausführungsbeispiel
- 50c: dorsales Fixationssystem nach einem dritten Ausführungsbeispiel
- 50d: dorsales Fixationssystem nach einem vierten Ausführungsbeispiel
- 50e: dorsales Fixationssystem nach einem fünften Ausführungsbeispiel
- 50f: dorsales Fixationssystem nach einem sechsten Ausführungsbeispiel
- 200: herkömmliche Haltevorrichtung
- α: erster Winkel zwischen dem Verbindungselement und dem ersten Pedikelstab
- β: zweiter Winkel zwischen dem Verbindungselement und dem zweiten Pedikelstab

## Patentansprüche

1. Haltevorrichtung (20a, 20b, 20c, 20d) für Wirbelkörper der Wirbelsäule aufweisend ein in einen Wirbelkörper einzubringendes Implantat (1) und einen Pedikelstab (2), wobei das Implantat (1) für die Beweglichkeit des Pedikelstabes (2) einen Hohlraum (3) aufweist, der Pedikelstab (2) in dem Hohlraum (3) für eine permanente Beweglichkeit des Pedikelstabes (2) in Bezug auf das Implantat (1) angeordnet ist und das Implantat (1) eine hülsenförmige Ausgestaltung aufweist **dadurch gekennzeichnet, dass** das Implantat (1) eine Gewindeverankerung (4) in dem Hohlraum (3) und der Pedikelstab (2) einen in die Gewindeverankerung (4) einbringbaren Gewindeabschnitt (5) aufweist, und das Implantat (1) posterior auf einer Seite einen Verlängerungsabschnitt (17) aufweist, der für eine dauerhafte Anbringung eines mit einer zweiten Haltevorrichtung (20a,, 20b, 20c, 20d) verbundenen Verbindungselements (11) mit einem Kopplungsmittel (18) ausgestaltet ist

2. Haltevorrichtung (20a, 20b, 20c, 20d) für Wirbelkörper nach Anspruch 1, **dadurch gekennzeichnet, dass** der Pedikelstab (2) einen elastischen Bereich aufweist.

3. Haltevorrichtung (20a, 20b, 20c, 20d) für Wirbelkörper nach Anspruch 2, **dadurch gekennzeichnet, dass** der elastische Bereich ein Material aufweist, das aus einer Gruppe ausgewählt worden ist, die umfasst: Metalle und insbesondere Titan und dessen Legierungen, Stahl, Nickel-Titan-Legierungen einschließlich Nitinol, Memory-metalle, oder CoCr oder Kunststoffe und insbesondere Polyethylen, Polyurethan, Polyetheretherketon, kohlenstofffaserverstärkten Kunststoff.

4. Haltevorrichtung (20a, 20b, 20c, 20d) für Wirbelkörper nach Anspruch 2 oder 3, **dadurch gekennzeichnet, dass** das Implantat (1) ein elastisches Element (10) aufweist, das an einem Verbindungsbereich zwischen dem Implantat (1) und dem Pedikelstab (2) angeordnet ist.

5. Haltevorrichtung (20a, 20b, 20c, 20d) für Wirbelkörper nach Anspruch 1, **dadurch gekennzeichnet, dass** eine einachsige oder eine mehrachsige Gelenkverbindung zwischen dem Implantat (1) und dem Pedikelstab (2) vorgesehen ist.

6. Haltevorrichtung (20a, 20b, 20c, 20d) für Wirbelkörper nach einem der vorhergehenden Ansprüche, **dadurch gekennzeichnet, dass** im posterioren Bereich das Implantat (1) eine Anschlagfläche (6) und der Pedikelstab (2) eine Anschlagsgegenfläche (7) zur Begrenzung der Beweglichkeit zwischen dem Implantat (1) und dem Pedikelstab (2) aufweisen.

7. Haltevorrichtung (20a, 20b, 20c, 20d) für Wirbelkörper nach einem der vorhergehenden Ansprüche, **dadurch gekennzeichnet, dass** der Pedikelstab (2) eine posterior schließbare Stabaufnahme (8) aufweist.

8. Haltevorrichtung (20a, 20b, 20c, 20d) für Wirbelkörper nach Anspruch 7, **dadurch gekennzeichnet, dass** die Stabaufnahme (8) ein verstellbares Sperrelement (9) umfasst.

9. Haltevorrichtung (20a, 20b, 20c, 20d) für Wirbelkörper nach einem der vorhergehenden Ansprüche, **dadurch gekennzeichnet, dass** der Pedikelstab (2) einen ovalen Querschnitt aufweist.

10. Haltevorrichtung (20a, 20b, 20c, 20d) für Wirbelkörper nach einem der vorhergehenden Ansprüche, **dadurch gekennzeichnet, dass** das Implantat (1) mit einer posterior abnehmenden Wandstärke ausgebildet ist.

11. Dorsales Fixationssystem (50b) mit einer Anzahl an Haltevorrichtungen (21, 22, 23) für Wirbelkörper nach einem der Ansprüche 1-10, **dadurch gekennzeichnet, dass** mindestens die erste Haltevorrichtung (21) ein Verbindungselement (11) aufweist, das über ein Befestigungsteil (15a) der ersten Haltevorrichtung (21) mit dem Pedikelstab (2a) der ersten Haltevorrichtung (21) und über das Kopplungsmittel (18b) der zweiten Haltevorrichtung (22) mit der zweiten Haltevorrichtung (22) verbunden ist.

12. Dorsales Fixationssystem (50a, 50c, 50d, 50e, 50f) mit einer ersten Haltevorrichtung (21) für Wirbelkörper nach einem der Ansprüche 1 bis 10 und einer zweiten Haltevorrichtung (22) für Wirbelkörper, **gekennzeichnet durch** ein Verbindungselement (11), das ein erstes Befestigungsteil (15a) mit dem ersten Pedikelstab (2a) der ersten Haltevorrichtung (21) und ein zweites Befestigungsteil (15b) mit dem zweiten Pedikelstab (2b) der zweiten Haltevorrichtung (22) aufweist.

13. Dorsales Fixationssystem (50a, 50c, 50d, 50e, 50f) nach Anspruch 11, **dadurch gekennzeichnet, dass** das erste Befestigungsteil (15a) und/oder das zweite Befestigungsteil (15b) ausgestaltet sind, einen ersten Winkel (α) zwischen dem Verbindungselement (11) und dem ersten Pedikelstab (2a) der ersten Haltevorrichtung (21) bzw. einen zweiten Winkel (β) zwischen dem Verbindungselement (11) und dem zweiten Pedikelstab (2b) der zweiten Haltevorrichtung (22) stabil zu halten.

14. Dorsales Fixationssystem (50a, 50c, 50d, 50e, 50f) nach Anspruch 11, **dadurch gekennzeichnet, dass** das erste Befestigungsteil (15a) und/oder das zweite Befestigungsteil (15b) ausgestaltet sind, einen ersten Winkel (α) zwischen dem Verbindungselement (11) und dem ersten Pedikelstab (2a) der ersten Haltevorrichtung (21) bzw. einen zweiten Winkel (β) zwischen dem Verbindungselement (11) und dem zweiten Pedikelstab (2b) der zweiten Haltevorrichtung (22) variabel zu halten.

15. Dorsales Fixationssystem (50a,50c, 50d, 50e) nach einem der Ansprüche 12 bis 14, **dadurch gekennzeichnet, dass** das Verbindungselement (11) einen teleskopischen Bereich (19) undloder einen flexiblen Bereich (30) aufweist.

16. Dorsales Fixationssystem (50a, 50c, 50d, 50e) nach einem der Ansprüche 14 bis 15, **dadurch gekennzeichnet, dass** es eine dritte Haltevorrichtung (23) für Wirbelkörper aufweist und dass das Verbindungselement (11) über ein drittes Befestigungsteil (15c) an der dritten Haltevorrichtung (23) mit einem dritten Pedikelstab (2c) der dritten Haltevorrichtung (23) verbunden ist.

## Claims

1. A holding device (20a, 20b, 20c, 20d) for spinal vertebrae of the spinal column having an implant (1) to be introduced into a vertebra and a pedicle rod (2), wherein the implant (1) has a cavity (3) for mobility of the pedicle rod (2), the pedicle rod (2) is arranged in the cavity (3) for permanent mobility of the pedicle rod (2) in relation to the implant (1), and the implant (1) is of a sleeve-shaped configuration, **characterised in that** the implant (1) has a threaded anchorage (4) in the cavity (3) and the pedicle rod (2) has a threaded portion (5) which can be introduced into the threaded anchorage (4), and the implant (1) has posteriorally on one side a prolongation portion (17) which is designed for permanent fitment of a connecting element (11) connected to a second holding device (20a, 20b, 20c, 20d) with a coupling means (18).

2. A holding device (20a, 20b, 20c, 20d) for spinal vertebrae according to claim 1 **characterised in that** the pedicle rod (2) has an elastic region.

3. A holding device (20a, 20b, 20c, 20d) for spinal vertebrae according to claim 2 **characterised in that** the elastic region has a material selected from a group which includes: metals and in particular titanium and alloys thereof, steel, nickel-titanium alloys including nitinol, memory metals, or CoCr or plastic materials and in particular polyethylene, polyurethane, polyetheretherketone and carbon fibre-reinforced plastic.

4. A holding device (20a, 20b, 20c, 20d) for spinal vertebrae according to claim 2 or claim 3 **characterised in that** the implant (1) has an elastic element (10) arranged at a connecting region between the implant (1) and the pedicle rod (2).

5. A holding device (20a, 20b, 20c, 20d) for spinal vertebrae according to claim 1 **characterised in that** there is provided a single-axis or multi-axis pivot connection between the implant (1) and the pedicle rod (2).

6. A holding device (20a, 20b, 20c, 20d) for spinal vertebrae according to one of the preceding claims **characterised in that** in the posterior region the implant (1) has an abutment surface (6) and the pedicle rod (2) has a counterpart abutment surface (7) for limiting the mobility between the implant (1) and the pedicle rod (2).

7. A holding device (20a, 20b, 20c, 20d) for spinal vertebrae according to one of the preceding claims **characterised in that** the pedicle rod (2) has a posteriorally closable rod receiving means (8).

8. A holding device (20a, 20b, 20c, 20d) for spinal vertebrae according to claim 7 **characterised in that** the rod receiving means (8) includes a displaceable closure element (9).

9. A holding device (20a, 20b, 20c, 20d) for spinal vertebrae according to one of the preceding claims **characterised in that** the pedicle rod (2) is of an oval cross-section.

10. A holding device (20a, 20b, 20c, 20d) for spinal vertebrae according to one of the preceding claims **characterised in that** the implant (1) is of a posteriorally decreasing wall thickness.

11. A dorsal fixing system (50b) having a number of holding devices (21, 22, 23) for spinal vertebrae according to one of claims 1 to 10, **characterised in that** at least the first holding device (21) has a connecting element (11) which is connected by way of a fixing portion (15a) of the first holding device (21) to the pedicle rod (2a) of the first holding device (21) and by way of the coupling means (18b) of the second holding device (22) to the second holding device (22).

12. A dorsal fixing system (50a, 50c, 50d, 50e, 50f) having a first holding device (21) for spinal vertebrae according to one of claims 1 to 10 and a second holding device (22) for spinal vertebrae, **characterised by** a connecting element (11) which has a first fixing portion (15a) with the first pedicle rod (2a) of the first holding device (21) and a second fixing portion (15b) with the second pedicle rod (2b) of the second holding device (22).

13. A dorsal fixing system (50a, 50c, 50d, 50e, 50f) according to claim 11 **characterised in that** the first fixing portion (15a) and/or the second fixing portion (15b) are adapted to hold stable a first angle (α) between the connecting element (11) and the first pedicle rod (2a) of the first holding device (21) and a second angle (β) between the connecting element (11) and the second pedicle rod (2b) of the second holding device (22) respectively.

14. A dorsal fixing system (50a, 50c, 50d, 50e, 50f) according to claim 11 **characterised in that** the first fixing portion (15a) and/or the second fixing portion (15b) are adapted to hold variably a first angle (α) between the connecting element (11) and the first pedicle rod (2a) of the first holding device (21) and a second angle (β) between the connecting element (11) and the second pedicle rod (2b) of the second holding device (22) respectively.

15. A dorsal fixing system (50a, 50c, 50d, 50e) according to one of claims 12 to 14 **characterised in that** the connecting element (11) has a telescopic region (19) and/or a flexible region (30).

16. A dorsal fixing system (50a, 50c, 50d, 50e) according to one of claims 14 to 15 **characterised in that** it has a third holding device (23) for spinal vertebrae and that the connecting element (11) is connected by way of a third fixing portion (15c) on the third holding device (23) to a third pedicle rod (2c) of the third holding device (23).

## Revendications

1. Dispositif de support (20a, 20b, 20c, 20d) destiné à des corps vertébraux de la colonne vertébrale, présentant un implant (1), devant être introduit dans un corps vertébral, et une tige pédiculaire (2), l'implant (1) présentant une cavité (3) pour la mobilité de la tige pédiculaire (2), la tige pédiculaire (2) étant placée dans la cavité (3) en vue d'une mobilité permanente de la tige pédiculaire (2) par rapport à l'implant (1), et l'implant (1) présentant un agencement en forme de manchon, **caractérisé en ce que** l'implant (1) présente un ancrage taraudé (4) dans la cavité (3) et la tige pédiculaire (2) présente une partie filetée (5) pouvant être introduite dans l'ancrage fileté (4), et l'implant (1) présente à l'arrière, sur un côté, une partie de prolongement (17) qui est agencée en vue d'un montage permanent d'un élément de liaison (11) relié à un deuxième dispositif de support (20a, 20b, 20c), avec un moyen d'accouplement (18).

2. Dispositif de support (20a, 20b, 20c, 20d) destiné à des corps vertébraux selon la revendication 1, **caractérisé en ce que** la tige pédiculaire (2) présente une zone élastique.

3. Dispositif de support (20a, 20b, 20c, 20d) destiné à des corps vertébraux selon la revendication 2, **caractérisé en ce que** la zone élastique présente un matériau qui a été sélectionné dans un groupe qui comprend : des métaux et en particulier le titane et ses alliages, l'acier, des alliages nickel-titane, y compris le nitinol, des métaux à mémoire de forme ou CoCr ou des matières plastiques et en particulier le polyéthylène, le polyuréthanne, le polyéther-éther-cétone, des matières plastiques renforcées aux fibres de carbone.

4. Dispositif de support (20a, 20b, 20c, 20d) destiné à des corps vertébraux selon la revendication 2 ou 3, **caractérisé en ce que** l'implant (1) présente un élément (10) élastique qui est disposé sur une zone de raccordement entre l'implant (1) et la tige pédiculaire (2).

5. Dispositif de support (20a, 20b, 20c, 20d) destiné à des corps vertébraux selon la revendication 1, **caractérisé en ce qu'**il est prévu une liaison articulé à un ou plusieurs axes entre l'implant (1) et la tige pédiculaire (2).

6. Dispositif de support (20a, 20b, 20c, 20d) destiné à des corps vertébraux selon l'une des revendications précédentes, **caractérisé en ce que** dans la zone postérieure, l'implant (1) présente une surface de butée (6), et la tige pédiculaire (2) présente une surface de butée antagoniste (7) pour la limiter la mobilité entre l'implant (1) et la tige pédiculaire (2).

7. Dispositif de support (20a, 20b, 20c, 20d) destiné à des corps vertébraux selon l'une des revendications précédentes, **caractérisé en ce que** la tige pédiculaire (2) présente un logement de tige (8) pouvant être fermé côté postérieur.

8. Dispositif de support (20a, 20b, 20c, 20d) destiné à des corps vertébraux selon la revendication 7, **caractérisé en ce que** le logement de tige (8) comprend un élément de blocage (9) réglable.

9. Dispositif de support (20a, 20b, 20c, 20d) destiné à des corps vertébraux selon l'une des revendications précédentes, **caractérisé en ce que** la tige pédiculaire (2) présente une section transversale ovale.

10. Dispositif de support (20a, 20b, 20c, 20d) destiné à des corps vertébraux selon l'une des revendications précédentes, **caractérisé en ce que** l'implant (1) est réalisé avec une épaisseur de paroi qui diminue côté postérieur.

11. Système de fixation dorsal (50b) comprenant un certain nombre de dispositifs de support (21, 22, 23) destinés à des corps vertébraux selon l'une des revendications 1 à 10, **caractérisé en ce qu**'au moins le premier dispositif de support (21) présente un élément de liaison (11) qui est relié par un élément de fixation (15a) du premier dispositif de support (21) à la tige pédiculaire (2) du premier dispositif de support (21) et est relié par le moyen d'accouplement (18b) du deuxième dispositif de support (22) au deuxième dispositif de support (22).

12. Système de fixation dorsal (50a, 50c, 50d, 50e, 50f) comprenant un premier dispositif de support (21) destiné à des corps vertébraux selon l'une des revendications 1 à 10 et un deuxième dispositif de support (22) destiné à des corps vertébraux, **caractérisé en ce qu'il** comporte un élément de liaison (11) qui présente un premier élément de fixation (15a) avec la première tige pédiculaire (2a) du premier dispositif de support (21) et un deuxième élément de fixation (15b) avec la deuxième tige pédiculaire (2b) du deuxième dispositif de support (22).

13. Système de fixation dorsal (50a, 50c, 50d, 50e, 50f) selon la revendication 11, **caractérisé en ce que** le premier élément de fixation (15a) et/ou le deuxième élément de fixation (15b) sont conçus pour maintenir stable un premier angle (α) entre l'élément de liaison (11) et la première tige pédiculaire (2a) du premier dispositif de support (21), respectivement un deuxième angle (β) entre l'élément de liaison (11) et la deuxième tige pédiculaire (2b) du deuxième dispositif de support (22).

14. Système de fixation dorsal (50a, 50c, 50d, 50e, 50f) selon la revendication 11, **caractérisé en ce que** le premier élément de fixation (15a) et/ou le deuxième élément de fixation (15b) sont conçus pour maintenir variable un premier angle (α) entre l'élément de liaison (11) et la première tige pédiculaire (2a) du premier dispositif de support (21), respectivement un deuxième angle (β) entre l'élément de liaison (11) et la deuxième tige pédiculaire (2b) du deuxième dispositif de support (22).

15. Système de fixation dorsal (50a, 50c, 50d, 50e) selon l'une des revendications 12 à 14, **caractérisé en ce que** l'élément de liaison (11) présente une zone télescopique (19) et/ou une zone flexible (30).

16. Système de fixation dorsal (50a, 50c, 50d, 50e) selon l'une des revendications 14 à 15, **caractérisé en ce qu**'il présente un troisième dispositif de support (23) destiné à des corps vertébraux, et en ce que l'élément de liaison (11) est relié par un troisième élément de fixation (15c) du troisième dispositif de support (23) à une troisième tige pédiculaire (2c) du troisième dispositif de support (23).
